# EUROPEAN PATENT APPLICATION

(11) **EP 3 299 012 A1**
(43) Date of publication of application: **28.03.2018**
(21) Application number: 16796550.8
(22) Date of filing: 19.05.2016
(51) Int. Cl.: A61K 9/10, A61K 31/13, A61K 31/445, A61K 47/10, A61K 47/18, A61K 47/24, A61P 43/00

(54) **TRANSDERMAL LIQUID PREPARATION**

(30) Priority: 19.05.2015 JP 2015101915; 19.08.2015 JP 2015162245; 07.10.2015 JP 2015199676; 17.12.2015 JP 2015246801; 26.02.2016 JP 2016035958
(71) Applicant: MEDRx Co., Ltd., Higashikagawa-shi Kagawa 769-2712 (JP)
(72) Inventor: YAMASAKI, Keiko, Higashikagawa-shi Kagawa 769-2712 (JP)
(74) Representative: Miller Sturt Kenyon
(86) International application number: PCT/JP2016/064826
(87) International publication number: WO 2016/186157

(57) **Abstract**

The present invention addresses the problem of providing a liquid preparation or external application, which has a shortened transdermal absorption lag time and has excellent transdermal absorption properties. The present invention also addresses the problem of providing a liquid preparation for external application, which is less irritative to skin. The problems can be solved by a transdermal liquid preparation comprising: (a) propylene glycol in an amount of 40 to 98% by weight; (b) phosphatidylcholine in an amount of 0.1 to 5% by weight; (c) oleyl alcohol and/or isostearyl alcohol in an amount of 0.1 to 10% by weight; and (d) a medicine in an amount of 0.1 to 25% by weight. It is preferred that the transdermal liquid preparation additionally contains an alkanol amine. The alkanol amine is preferably triethanolamine.

## Description

### Technical Field

The present invention relates to a transdermal absorptive liquid formulation. Specifically, the present invention relates to a composition for external preparation comprising a medicament and phosphatidylcholine.

### Background Art

A transdermal absorptive liquid formulation in which a medicament and phosphatidylcholine disperse in propylene glycol is known as a technology allowing various medicaments to be absorbed transdermally (Patent document 1). The technology exhibits an excellent effect in improving the transdermal permeability for various medicaments with a simple composition. However, some medicaments are highly desired to be absorbed in a very short time, thus further improvement in percutaneous permeability has been required.

On the other hand, it is proposed that a transdermal absorption accelerator such as higher fatty acids, higher alcohols, and fatty acid esters is added in order to improve transdermal absorbability (for example, Patent Documents 2 and 3). However a technology to shorten a transdermal absorption lag time and to improve transdermal absorbability of colloidal liquid formulation containing propylene glycol and phosphatidylcholine has not been proposed.

### Prior Art Document

### Patent Document

Patent Document 1: WO2015/072564
Patent Document 2: Japanese Patent Publication No 2014-152162
Patent Document 3: Japanese Patent Publication No 2013-60395

### Summary of the Invention

### Problems to be solved by the Invention

An object of the present invention is to provide a transdermal absorptive liquid formulation having a shortened transdermal absorption lag time and capable of reaching a maximum skin permeation rate smoothly. Another object of the present invention is to provide a transdermal absorptive liquid formulation which has a reduced skin irritation and is suitable for long-term and/or prolonged administration.

### Means for solving the Problems

The present inventor found a transdermal absorptive liquid formulation to have a transdermal absorption lag time shortened and capable of reaching a high drug concentration in the plasma in a short time by adding a specific higher alcohol to a colloidal dispersion liquid comprising a medicament, phosphatidylcholine and propylene glycol, and thus completed the present invention.

Namely, the present invention provides a transdermal absorptive liquid formulation comprising
(a) 40 to 98 weight % of propylene glycol,
(b) 0.1 to 5 weight % of phosphatidylcholine,
(c) 0.1 to 10 weight % of oleyl alcohol and/or isostearyl alcohol, and
(d) 0.1 to 25 weight % of a medicament.

### Effect of the Invention

The present invention can provide a transdermal absorptive liquid formulation having a short transdermal absorption lag time and capable of reaching a maximum skin permeation rate smoothly. Furthermore, the present invention can provide a transdermal absorptive liquid formulation of which skin irritation is significantly reduced even if it is a medicament with which skin irritation due to increase of skin permeation rate in conventional transdermal administration formulations has been reported, and suitable for long-term and/or prolonged administration.

### Brief explanation of the drawings

[Fig.1] Fig.1 is a graph showing the result of the in vivo rizatriptan plasma concentration evaluation test of the liquid formulation of Example 1-1 and Comparative example 1-1 using rats.
[Fig.2] Fig.2 is a graph showing the result of the in vivo donepezil plasma concentration evaluation test of the liquid formulation of Example 1-1 and Comparative example 1-3 using miniature pigs.
[Fig.3] Fig.3 is a graph showing the result of the in vitro skin permeability test of the liquid formulation of Example 4 using rat skin.

### Mode for Carrying out the Invention

The transdermal absorptive liquid formulation of the present invention contains a medicament and phosphatidylcholine in propylene glycol or propylene glycol-containing solvent and further contains oleyl alcohol and/or isostearyl alcohol (16-methylheptadecene-1-ol) . The "propylene glycol-containing solvent" in the present specification denotes propylene glycol in which a solubilizing agent such as water and /or a hydrophilic solvent is dissolved and mixed with as described below.

As the "medicament", any of low-molecule, middle-molecule, and high-molecule compounds having a physiological activity, which usually have one or more polar groups, can be utilized. The term "medicament" in the present specification includes a pharmaceutically acceptable salt thereof. The above-mentioned "low-molecule compound" means compounds of which the molecular weight is less than about 500. Specifically, a medicament having indane structure such as ramelteon, rasagiline, and donepezil; a medicament having indole structure such as rizatriptan, sumatriptan, naratriptan, zolmitriptan, eletriptan, almotriptan; a medicament having piperidine skeleton such as donepezil, trihexyphenidyl, perisoxal; a medicament having benzofuran skeleton such as morphine, oxycodone, galantamine; a medicament having isobenzofuran structure such as escitalopram, galantamine; a medicament having benzodioxole structure such as paroxetine; a medicament having indole structure such as indometacin, pergolide, bromocriptine, ondansetron; a medicament having indazole structure such as granisetron; a medicament having benzothiophen structure such as raloxifene, zileuton; a medicament having benzoisothiazole structure such as lurasidone; a medicament having benzothiadiazole structure such as tizanidine; a medicament having adamantane skeleton such as amantadine, memantine, vildagliptin; an opioid pain reliever such as tramadol, morphine, oxycodone, hydromorphone; a histidine derivative such as anserine; a salicylic acid derivative such as aspirin, methyl salicylate can be exemplified. The above-mentioned "middle-molecule compound" means compounds of which the molecular weight is about 500 to 2,000, and can include peptides. The above-mentioned "high-molecule compound" means compounds of which the molecular weight is about 150,000 to 2,000,000, and a sugar chain and a protein, etc. can be exemplified. The medicament can be used in a combination of two or more thereof.

The medicament can be used alone or in a combination of two or more thereof. The contents of the medicament can be selected from, for example, the range of from 0.1 to 25 weight % relative to the total weight of the liquid formulation.

Phosphatidylcholine is a collective term of a compound represented by formula (I), and usually be provided as a mixture wherein the kinds or combinations of R¹ and R² are different. 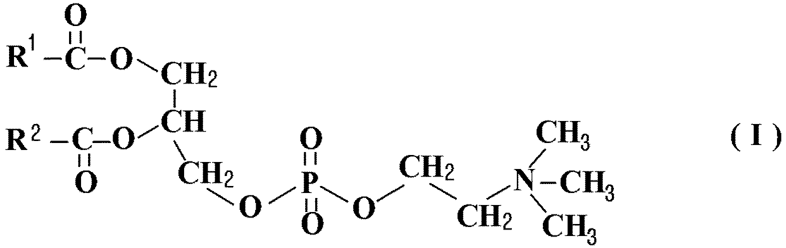 (In the formula, R¹ and R² are the same or different each other, and each is a C₁₂₋₂₂ hydrocarbon group.)

An unsaturated phosphatidylcholine in which at least one of R¹ and R² is an unsaturated hydrocarbon group can be utilized in the present invention. Though it may be possible that a saturated hydrocarbon group such as palmityl group (16:0), stearyl group (18:0) is included as R¹ and R², in the unsaturated phosphatidylcholine which can be utilized in the present invention, the content rate of the saturated hydrocarbon group is less than 80%, preferably less than 70%, more preferably less than 60%, particularly preferably less than 50%. Palmitoyl group (16:1), oleyl group (18:1), linoleyl group (18:2), linolenyl group (18:3) can be exemplified as the unsaturated hydrocarbon group. It is preferred that the content rate of the unsaturated hydrocarbon group having 18 carbon atoms such as oleyl group, linoleyl group, linolenyl group, and the like is 20% or more, more preferably 30% or more, particularly preferably 40% or more, in the unsaturated phosphatidylcholine which can be utilized in the present invention. With the use of the unsaturated phosphatidylcholine, a stable colloidal dispersion with excellent transdermal permeability can be prepared.

In the present invention, a highly purified phosphatidylcholine of natural product origin such as a soybean lecithin, an egg yolk lecithin and the like, and whose content rate of the phosphatidylcholine is 95% or more , can be used preferably. It is not preferable to use a chemically and/or biologically modified phosphatidylcholine such as a hydrogenated phosphatidylcholine obtained by hydrogenation treatment, or a lysophosphatidylcholine obtained by enzymatic treatment or the like, because a stable colloidal dispersion may not be obtained. However, of the chemically or biologically modified phosphatidylcholine such as a partially hydrogenated product of a naturally derived lecithin, a highly purified phosphatidylcholine having a high unsaturation degree (for example, the iodine value is 20 or more, and the content rate of lysolecithin is less than 10%) can be utilized as the "unsaturated phosphatidylcholine" in the present invention.

The content of phosphatidylcholine is selected from, usually, the range of 0.1 to 5 w/w%, preferably 0.1 to 3 w/w%, more preferably 0.1 to 2.0 w/w%, particularly preferably 0.2 to 1.5 w/w%. It is not preferable that the content of phosphatidylcholine is less than 0.1 w/w%, because a stable liquid formulation may not be formed. Adding phosphatidylcholine over 5 w/w% does not cause the improvement of transdermal permeability depending on the increase in the phosphatidylcholine content.

The liquid formulation of the present invention is prepared by mixing a medicament dissolved in propylene glycol with phosphatidylcholine dissolved in propylene glycol. If the medicament is insoluble or poorly-soluble in propylene glycol, and so a desired quantity cannot be dissolved, a solubilizing agent such as water, polyethylene glycol can be added to form "propylene glycol-containing solvent". The medicament of dissolved state can be prepared by dissolving the medicament in the propylene glycol-containing solvent. Adding phosphatidylcholine or propylene glycol solution of phosphatidylcholine to the medicament dissolved in propylene glycol or the propylene glycol-containing solvent, followed by mixing can provide a liquid formulation with excellent transdermal permeability. The liquid formulation is usually a colloidal dispersed liquid. If the medicament is a low molecule, the median particle size is observed within the range of from 5 nm to 200 nm in many cases. The content of the solubilizing agent can be selected from a range in which ratio of propylene glycol to total weight of the liquid formulation is 40 weight% or more, preferably 60 weight% or more. The content of the solubilizing agent to propylene glycol can be selected, for example, from the range of 0 to 50 weight%, preferably from the range of 0 to 35 weight%.

The propylene glycol-containing solvent can further contain a hydrophilic organic solvent miscible with propylene glycol if needed. Specifically, a polyhydric alcohol such as glycerin, 1,3-butandiol can be exemplified. The content of the hydrophilic organic solvent is, for example less than 30 weight% of the propylene glycol-containing solvent, preferably less than 20 weight%. The content of the hydrophilic organic solvent relative to propylene glycol is less than 50 weight% of propylene glycol.

The liquid formulation of the present invention further contains oleyl alcohol and/or isostearyl alcohol. Containing oleyl alcohol and/or isostearyl alcohol remarkably improves the transdermal permeability of a medicament. Especially, the transdermal absorption lag time is shortened to reach the maximum skin permeation rate smoothly. The addition of oleyl alcohol and/or isostearyl alcohol is selected from, for example, 0.1 to 10 weight%, preferably 0.2 to 5 weight%, relative to the total weight of the liquid formulation. If the addition of oleyl alcohol and/or isostearyl alcohol is less than 0.1 weight%, it may be hard to obtain transdermal permeation promoting effect. Even if oleyl alcohol and/or isostearyl alcohol is added more than 5 weight%, the improvement of transdermal permeability depending on concentration is not be observed.

Both oleyl alcohol and isostearyl alcohol are effective in improving the transdermal permeation in combination with the liquid formulation containing phosphatidylcholine and propylene glycol, and especially oleyl alcohol is highly effective. Therefore, it is preferred to contain at least oleyl alcohol. When oleyl alcohol and isostearyl alcohol are used jointly, their mixing ration is not especially limited, and can be selected arbitrarily.

The liquid formulation of the present invention preferably further contains an alkanolamine. Containing the alkanolamine further improves the transdermal permeability of a medicament. Any of primary, secondary or tertiary alkanolamines having 2 to 12 carbon atoms can be utilized. Among them, the secondary or tertiary alkanolamines are preferred, and the tertiary alkanolamines are especially preferred. Specifically, diethanolamine, triethanolamine, diisopropanolamine, triisopropanolamine are exemplified. Triethanolamine is notably preferred because it is superior in an effect of accelerating transdermal permeation.

The content of alkanolamine is selected from the range of 0.01 to 10 w/w%, depending on the properties of a medicament. Most of the medicaments remarkably exhibit the transdermal permeability promoting effect in the range of 0.1 to 5 w/w%, particularly 0.2 to 2 w/w%. In case that the medicament is donepezil hydrochloride, the addition of alkanolamine is preferably 0.01 to 1.0 w/w% because the addition of alkanolamine exceeding 1 w/w% may cause precipitation or aggregation of crystal.

The liquid formulation of the present invention can also contain various additives used for an external preparation or a cosmetic if needed. As the additives, a fragrance, an antioxidant agent, an antiseptic, colorant, buffer, a pH adjuster, an ultraviolet absorber, an antibacterial medicine, and the like can be exemplified. As the antioxidant agent, tocopherol acetate, edetate sodium, erythorbic acid, 1, 3-butylene glycol, sodium pyrosulfite and the like can be exemplified. As the antiseptic, sorbic acid, taurine, and the like are exemplified. As the pH adjuster, organic acids such as citric acid, acetic acid, and tartaric acid; inorganic acids such as phosphoric acid, and hydrochloric acid, can be exemplified.

The method for applying the liquid formulation of the present invention for the skin is not particularly restricted, and the method of applying or spraying the liquid formulation, the method of attaching a suitable carrier carrying the liquid formulation to the skin can be exemplified. Among them, the method of attaching the carrier (a nonwoven fabric, a foamed matrix, etc.) carrying the liquid formulation is preferable in easy adjustment of the dosage or operatability. Examples

### [Liquid formulation containing rizatriptan]

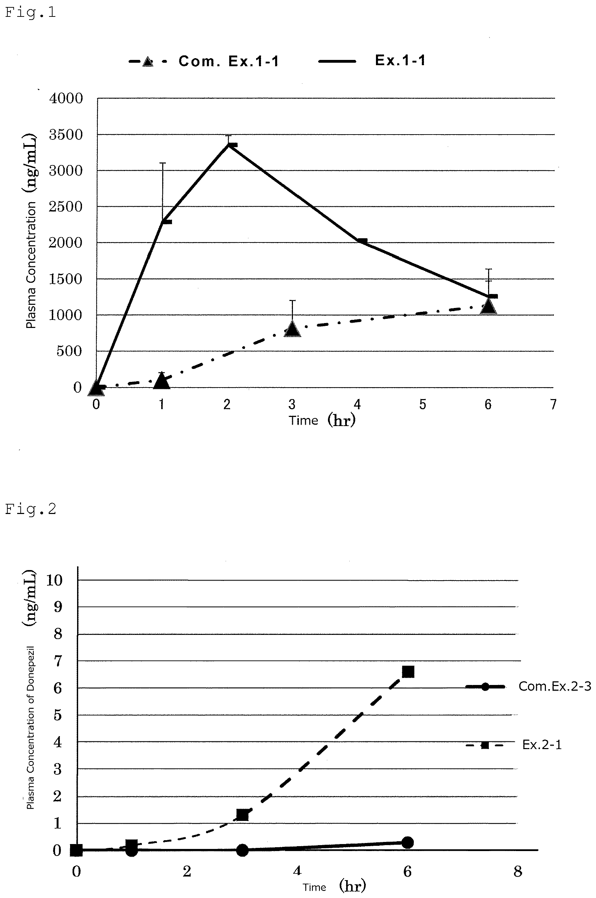

The liquid formulations of the compositions (w/w%) shown in Table 1 were prepared. In vivo plasma concentration using rats was evaluated for the obtained liquid formulation. Plasma concentrations of rizatriptan (ng/ml) at each blood collection point are shown in Table 1 as well. Transition of plasma concentration is shown in Figure 1.
The testing condition was as follows;
Animal Species : hairless rat (HWY/Slc) 5 weeks old
Dosage Amount : Example 1-1, medicament liquid 0.06 g/2 cm² (rizatriptan 12 mg)

### Comparative Example 1-1, medicament liquid 0.27 g/9cm² (rizatriptan 27 mg)

Measuring Means : HPLC

**Table 1**

| | | Ex.1-1 | Com.1-1 |
|---|---|---|---|
| Rizatriptan | | 20 | 10 |
| Propylene glycol | | 76.5 | 84.5 |
| Phosphatidylcholine | | 0.5 | 0.5 |
| Oleyl alcohol | | 2.5 | 0 |
| triethanolamine | | 0.5 | 5 |
| total | | 100 | 100 |
| Plasma concentration of rizatriptan (ng/ml) | 1 hour later | 2286 | 99.8 |
| | 2 hours later | 3353 | - |
| | 3 hours later | - | 817.3 |
| | 4 hours later | 2027 | - |
| | 6 hours later | 1258 | 1135 |

In the liquid formulation of the present invention containing oleyl alcohol, rizatriptan transitioned to plasma rapidly, and the plasma concentration reached its peak after 2 hours. In the liquid formulation of the comparative example which does not contain oleyl alcohol, only a small amount of the medicament transitioned to plasma despite the adhering area was broad, and the dosage amount was large. Moreover, its transdermal penetration lag time was long.

### [Liquid formulation containing memantine and donepezil]

The liquid formulations of the compositions (weight %) shown in Table 2 were prepared. Transdermal permeability of donepezil and memantine in the obtained liquid formulation was evaluated with the use of Franz cell. In the test, the skin of Yucatan micro pig (male 5 months old) was used. The receptor solution was a (water : ethanol =9 :1)-solution. Cumulative skin permeation amounts (µg/cm²) after 7 hours are shown in Table 2.

In vivo plasma concentration was evaluated for the liquid preparation obtained in Example 2-1 and Comparative Example 2-3 using miniature pigs. Transition of the plasma concentration is shown in Fig 2.
The testing condition was as follows;
Animal Species : miniature pig
Dosage Amount : Example 1-1, medicament liquid 6 g/100cm² (donepezil 300 mg)

### Comparative Example 2-3, medicament liquid 6 g/100cm² (donepezil 300 mg)

Measuring Means : HPLC

**Table 2**

| | | Ex. 2-1 | Ex. 2-2 | Ex. 3-5 | Com. 2-1 | Com. 2-2 | Com. 2-3 |
|---|---|---|---|---|---|---|---|
| Donepezil hydrochloride | | 5.0 | 5.5 | - | 5.5 | 5.5 | 5.5 |
| Memantine hydrochloride | | 10.0 | 6.0 | 6.0 | 6.0 | 6.0 | - |
| Propylene glycol | | 68.2 | 50.0 | 72.0 | 69.5 | 66.7 | 78.1 |
| 1,3-butanediol | | - | - | 16.6 | - | - | - |
| Phosphatidylcholine | | 0.2 | 0.3 | 0.5 | 0. 7 | - | 0.8 |
| triethanolamine | | 0.8 | 0.8 | 0.4 | 1.3 | 1.0 | 0.6 |
| Purified water | | 15.0 | 5.0 | 3 | 17 | 20 | 15.0 |
| Oleyl alcohol | | 0.8 | - | 1.5 | - | 0.8 | - |
| Isostearyl alcohol | | - | 2.4 | - | - | - | - |
| Total | | 100 | 100 | 100 | 100 | 100 | 100 |
| Cumulative skin permeation amount after 6hr | Donepezil | 9.7 | 8.4 | | 1.4 | 3.1 | 0.5 |
| | memantine | 86.6 | 47.7 | 43.2 | 4.1 | 9.3 | |

It was confirmed that the liquid formulation of Example 2-1 containing oleyl alcohol and the liquid formulation of Example 2-2 containing isostearyl alcohol increased transdermal permeability in a short time of 7 hours, compared to the liquid formulation of Comparative Example 2-1 containing none of oleyl alcohol and isostearyl alcohol and the liquid formulation of Comparative Example 2-2 containing oleyl alcohol without phosphatidylcholine. The combination of a higher alcohol and phosphatidylcholine generated a synergy effect, that is, significantly improved the transdermal permeability in a short time.

### [Liquid formulation containing memantine]

The liquid formulations of the compositions (weight %) shown in Table 3 were prepared. Transdermal permeability of memantine in the obtained liquid formulation was evaluated with the use of Franz cell. In the test, the excised abdominal skin of 5-weeks old hairless rat (male) was used. The receptor solution was a (water : ethanol =9 :1)-solution. Cumulative skin permeation amounts (µg/cm²) after 6 hours are shown in Table 3.

**Table 3**

| | Ex. 3-1 | Ex. 3-2 | Ex. 3-3 | Ex. 3-4 | Ex. 3-5 | Com . |
|---|---|---|---|---|---|---|
| Memantine hydrochloride | 5.0 | 5.0 | 5.0 | 5.0 | 6.0 | 5.0 |
| Phosphatidylcholine | 0.1 | 0.25 | 0.5 | 1.0 | 0.5 | 0 |
| Propylene glycol | 87.1 | 86.95 | 86.7 | 86.2 | | |
| 1,3-butanediol | - | - | - | - | 16.6 | - |
| triethanolamine | 0.4 | 0.4 | 0.4 | 0.4 | 0.4 | 0.4 |
| Isostearyl alcohol | 2.4 | 2.4 | 2.4 | 2.4 | - | 2.4 |
| Oleyl alcohol | - | - | - | - | 1.5 | - |
| water | 5 | 5 | 5 | 5 | 3 | 5 |
| Total | 100 | 100 | 100 | 100 | 100 | 100 |
| Cumulative skin permeation amount after 6hr | 1048 | 1484 | 1600 | 1455 | 1927 | 358 |
| (µg/cm²) | | | | | | |

The liquid formulation of Example 3-1 to Example 3-5 containing both phosphatidylcholine and isostearyl or oleyl alcohol exhibited excellent transdermal permeability in the vitro test. Therefore, it was estimated that memantine should be also permeated through the skin in skin irritation study in rabbits. However, the skin irritation was minor. In contrast, the liquid formulation of Comparative Example 3-1 without phosphatidylcholine caused a strong skin irritation relative to the liquid formulations of Examples 3-1 to 3-5, despite they showed lower permeability.

### [Comparative formulation]

A publicly known transdermal preparation containing memantine (described in Japanese Patent Publication No. 2009-13171) was prepared in a manner as follows;

| | |
|---|---|
| Memantine hydrochloride | 20.0% |
| Sodium hydroxide | 3.7% |
| An acrylic adhesive having hydroxyl group | 76.3% |

Aqueous solution of sodium hydroxide was added to and mixed with memantine hydrochloride which was weighted at the above ratio. Acrylic adhesive having hydroxyl group was added to it, and was coated on a support in about 100 µm thickness, and dried to produce a patch preparation.

### [In vitro transdermal permeability test using human skin / Primary skin irritation test in rabbits]

The liquid formulation of Example 3-6 according to the formulation shown in Table 4 was prepared. The in vitro transdermal permeability test using human skin was conducted for the liquid formulation of Example 3-6 and the comparative formulation

In the skin irritation test, a piece of urethane form impregnated with the liquid formulation of Example 3-6 (0.03 g/cm²) was used as a test preparation. They were applied on rabbits (Kbl : JW male, 8 weeks old) for 24 hours. Skin conditions were observed visually at 1, 24, and 48 hours later after the removal of test preparations, and the primary skin irritation was evaluated by the Draize test. Primary irritation index (P.I.I.) is shown in Table 4 as well.

**Table 4**

| | Ex.3-6 | Com. |
|---|---|---|
| Memantine hydrochloride | 5.0 | |
| Phosphatidylcholine | 0.42 | |
| Propylene glycol | 87.3 | |
| triethanolamine | 0.4 | |
| Isostearyl alcohol | 1.2 | |
| Oleyl alcohol | 0.68 | |
| water | 5 | |
| Total | 100 | |
| cumulative human skin permeation amount (µg/cm²) | 991.8 | 529.7 |
| P.I.I. | 0.7 | 2 |

It was considered that memantine was permeated through skin in the skin irritation test in rabbits, because the liquid formulation of Example 3-6 exhibited excellent transdermal permeability in the vitro test. However the skin irritation was slight.

### [Liquid formulation containing tramadol]

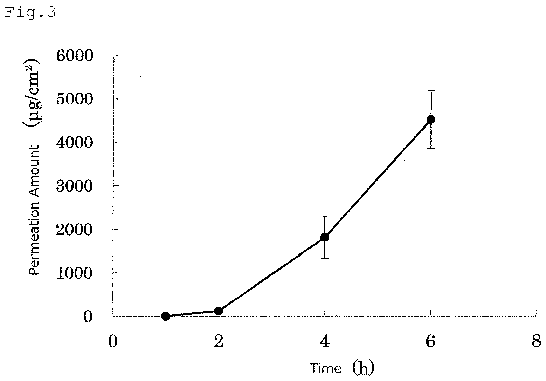

The liquid formulation of the composition (weight %) shown in Table 5 was prepared. Transdermal permeability of tramadol in the obtained liquid formulation was evaluated with the use of Franz cell. In the test, the abdominal excised skin of 5-weeks old hairless rat was used. The receptor solution was a (water : ethanol=9:1)-solution. Cumulative skin permeation amount (µg/cm²) for 6 hours from the start of the test is shown in Table 5 as well. Transition of the cumulative skin permeation amount is shown in Figure 3.

A piece of urethane form impregnated with the obtained liquid formulation (2×2 cm² : 0.03 g/cm²) was applied on rabbits (Kbl : JW male, 8 weeks old) for 24 hours. Skin conditions were observed visually at 1, 24, and 48 hours later after the removal of test preparations, and the primary skin irritation was evaluated by the Draize test. Primary irritation index (P.I.I.) is shown in Table 5 as well.

**Table 5**

| | | Ex.5 |
|---|---|---|
| Tramadol hydrochloride | | 10 |
| Phosphatidylcholine | | 0.5 |
| Propylene glycol | | 85.5 |
| triethanolamine | | 1.5 |
| Oleyl alcohol | | 1 |
| water | | 1.5 |
| total | | 100 |
| cumulative skin permeation amount (µg/cm²) | 4hr | 1815.6 |
| | 6hr | 4523.4 |
| P.I.I. | | 0 |

The liquid formulation of the present invention containing tramadol showed extremely high permeability in a short time: 1815.6 µg/cm² in 4 hours, 4523.4 µg/cm² in 6 hours. Further, skin irritation was not confirmed in the primary skin irritation test in rabbits.

### [Reference Preparation Example]

The liquid formulations of the compositions shown in Table 6 were prepared. The transdermal permeability of donepezil and memantine in the obtained liquid formulations was evaluated with the use of Franz cell. In the test, the skin of Yucatan micro pig (male 5 months old) was used. The receptor solution was a (water : ethanol =9 :1)-solution. Cumulative skin permeation amounts (µg/cm²) after 6 or 24 hours are shown in Table 6.

**Table 6**

| | | Liquid a | Liquid b |
|---|---|---|---|
| donepezil hydrochloride | | 5.5 | 5.5 |
| Memantine hydrochloride | | 6.0 | - |
| Propylene glycol | | 68.9 | 78.1 |
| Phosphatidylcholine | | 0.6 | 0.8 |
| triethanolamine | | 1.0 | 0.6 |
| water | | 18.0 | 15.0 |
| total | | 100 | 100 |
| cumulative skin permeation amount 24hr (µg/cm²) | donepezil | 70.4 | 13.1 |
| | memantine | 262.8 | - |

"Liquid a" showed a skin permeation amount of donepezil significantly greater than that of "Liquid b" without memantine. The reason was assumed that memantine accelerated transdermal permeation of donepezil. On the other hand, presence or absence of donepezil did not affect the skin permeation amount of memantine.

## Claims

1. A transdermal absorptive liquid formulation comprising
(a) 40 to 98 weight % of propylene glycol,
(b) 0.1 to 5 weight % of phosphatidylcholine,
(c) 0.1 to 10 weight % of oleyl alcohol and/or isostearyl alcohol, and
(d) 0.1 to 25 weight % of a medicament.

2. The transdermal absorptive liquid formulation according to claim 1, further comprising an alkanolamine.
